# EUROPEAN PATENT APPLICATION

(11) **EP 4 685 224 A2**
(43) Date of publication of application: **28.01.2026**
(21) Application number: 25209575.7
(22) Date of filing: 14.06.2017
(51) Int. Cl.: C12M 1/34

(54) **LEVEL AND/OR DENSITY SENSOR DEVICE FOR LIQUID VESSELS**

(30) Priority: 14.06.2016 PT 2016109451
(62) Divisional of application: 17742854.7
(71) Applicant: Watgrid, S.A., 3830-352 Ilhavo (PT)
(72) Inventor: PATRÍCIO DOMINGUES GONÇALVES, Fábio, Santiago de Litém (PT); BOTAS BILRO, Lúcia Maria, Gafanha da Encarnação (PT); NUNES NOGUEIRA, Rogério, Gafanha da Boa Hora (PT)
(74) Representative: Schmidt, Christian

(57) **Abstract**

The present disclosure is related to a level and/or density sensor for vessels suitable for storing liquids, in particular barrels or vats, more in particular barrels or vats for storing or producing wine. The hydrostatic pressure differential sensor for measuring volume and/or density disclosed herein comprises a main body; a tube for diving into the liquid; a hydrostatic pressure differential sensor; an air injector for injecting air into said tube; wherein the tube is coupled to the main body; wherein the hydrostatic pressure differential sensor has two inlets, a first inlet airtight connected to the upper top of said tube, configured so that the tube maintains air present in the interior thereof when dived into the liquid, and a second inlet for communicating with the interior of the vessel.

## Description

### Technical field

The present disclosure is related to a level and density sensor device for vessels suitable for storing liquids, in particular barrels or vats, more in particular barrels or vats for storing or producing wine.

### Background

The wine-making process is a set of technical operations allowing the transformation of grapes into wine and comprising several steps among which wine fermentation, ageing and storage steps in barrels/tuns/casks/vats or other vessels suitable for the purpose.

Offline control by sampling means is the most common form of controlling the wine-making process, namely for controlling the density.

The control of the wine level, when stored in barrels, normally implies opening the barrel, removing the stopper, for verifying the existing space between the top of the barrel and the liquid level.

Controlling the level is essential for ullage, which consists of resetting the level for compensating the losses occurred through gaseous exchange, which usually occurs every 2/3 months. It is estimated that each barrel loses 7 to 11 litres of wine, depending on the environmental conditions.

Gaseous exchanges are directly related to the wine oxygen dissolution, with various redox reactions occurring, which will form unstable compounds, leading to changes in colour and wine astringency and contributing to the ethanol oxidation to acetaldehyde, being that the acetaldehyde contributes afterwards to the copolymerisation of the flavonoids and anthocyanins.

The existence of an excessive air pocket promotes wine oxidation. Therefore, the wine level control is related to the quantity of available oxygen.

In the alcoholic fermentation step (transformation of sugars into alcohol) measuring the liquid density (must) is one of the ways of controlling the activity of yeasts, allowing for accompanying the fermentation progress.

These facts are described so as to illustrate the technical problem solved by the embodiments of this document.

### General description

The present disclosure is related to a level and/or density sensor device for vessels suitable for storing liquids, in particular barrels or vats, more in particular barrels or vats for fermenting, storing and producing wine.

Offline control by sampling means is the most common way of controlling the wine-making process. However, this way of control, by sampling, translates into a process that:
must be carried out by an operator on the wine storage location;
consumes time and resources for wine producers, since it is a manual process;
induces an action/reaction cycle causing greater variations, which leads to a lower wine quality;
is carried out only for a fraction of the production and consequently, the product quality is not uniform.

The importance of knowing at every moment what is the level and density of the liquid contained in a cask/tun/barrel/vat, relates to the following aspects:

| **Variable of interest** | **Barrel** | **Fermentation and/or storage reservoir** |
|---|---|---|
| **Level** | **Wine** - At the ageing stage it allows accompanying the evaporation, which is directly related to the gaseous exchanges with the exterior, allowing for evaluating the need to carry out ullage. | It may detect the existence of leakage, unusual liquid evaporation and stock control. |
| | **Spirit drinks** - At the ageing stage it allows accompanying the evaporation, which is directly related to the alcohol content of the spirit drink. | |
| **Density** | **Wine** - It allows accompanying it in the fermentation stage, that is, the sugar degradation, being that the yeast activity is followed so as to transform sugar into alcohol (in the fermentation stage density may go from 1100 kg/m³ to 990 kg/m³ at a temperature of 20 °C). | **Wine** - It allows accompanying it in the fermentation stage, that is, the sugar degradation. |
| | **Spirit drinks** - In the ageing stage it allows accompanying the reduction of the alcohol content. | |

Controlling the level and density of the liquid contained in a cask/tun/barrel/vat is a complex and lengthy process for being carried out manually, considering, for example, that each barrel/vat may contain a different liquid, or be in a fermentation/ageing stage different than the remaining barrels/vats, that is, there may exist a plurality of different needs for the various barrels/vats. Therefore, this problem is solved with the present disclosure through the implementation of the sensor device disclosed herein in each barrel/vat.

Solutions currently existing in the market present the following limitations: don't allow for an automated/in real time monitoring (buoy solution); present a high cost reducing economic viability; are affected by the deposition of sediments/particles (crystallisation effects), needing a regular maintenance (capacitive/pressure sensors with membranes in direct contact with the liquid); operation ranges outside the necessary requirements (ultrasounds distances> 300mm) and insufficient resolutions (>1mm ultrasounds).

The advantages of the present disclosure comprise:
increasing the wine quality;
reducing waste, since the sampling need for chemical analyses and additionally losses from meagre monitoring of the quality, possibly leading to the total loss of the product;
reducing control costs;
possibility of controlling a set of barrels through a network of sensor devices incorporated in a monitoring platform, which translates into control and monitoring in real time;
the physical and chemical integrity of the liquid is not endangered (there is no contamination);
facilitating the maintenance since the particle deposition does not compromise the functioning of the sensor device.

In an embodiment, it is possible to control the following parameters in real time: level, density, temperature, pH, turbidity, colour, sugar and alcohol quantity contained in the vessel/barrel/tun/cask/vat, namely through the use of additional sensors.

Embodiments of the present disclosure comprise the possibility of controlling the various parameters aforementioned more than once; sending notifications when the monitored parameters are outside the limits defined by the user; real time monitoring; wireless communication; detecting anomalies, detecting typical profiles, comparing history data, data mining, big data algorithms, anticipating/forecasting maintenance tasks, production management, increasing energy efficiency.

The present disclosure allows measuring the hydrostatic pressure of a liquid contained, for example, in a barrel without having to open it, since the variation of the hydrostatic pressure between two points, one situated on the free surface of the liquid and another situated in the point from which the measurement is intended to be carried out (level measurement). Therefore, avoiding opening the barrel also translates into the following advantages: avoiding excessive gaseous exchanges leading to the excessive dissolution of oxygen leading to the formation of compounds, resulting from redox reactions, increasing the risk of loss of the quality potential or even the total loss of the product.

The present disclosure is related to the hydrostatic pressure, that is, it is related to the forces exerted by and on fluids at rest and relates to a level or density sensor device for barrels/sensorised stopper for barrels or vats, in particular a differential pressure sensor allowing for determining the level of a liquid contained in a vessel suitable for storing liquids, in particular, wine and also allowing for determining the density of said liquid contained in the vessel/barrel.

The hydrostatic pressure is determined by Stevin's theorem. The operating principle of the level measurement by the sensorised stopper is based on Stevin's theorem: *P = ρgh* wherein ***P*** corresponds to the hydrostatic pressure created by a column of liquid, ***ρ*** corresponds to the density of the liquid in kg/m³, ***g*** corresponds to the gravity acceleration in m/s², ***h*** corresponds to the liquid height/level above the measure point in metres. By measuring the value of ***P*** and considering that the value of ***ρ*** and **g** are constant after the calibration has been made, it is possible to obtain the value of ***h*,** which in this case represents the level of the liquid inside the barrel.

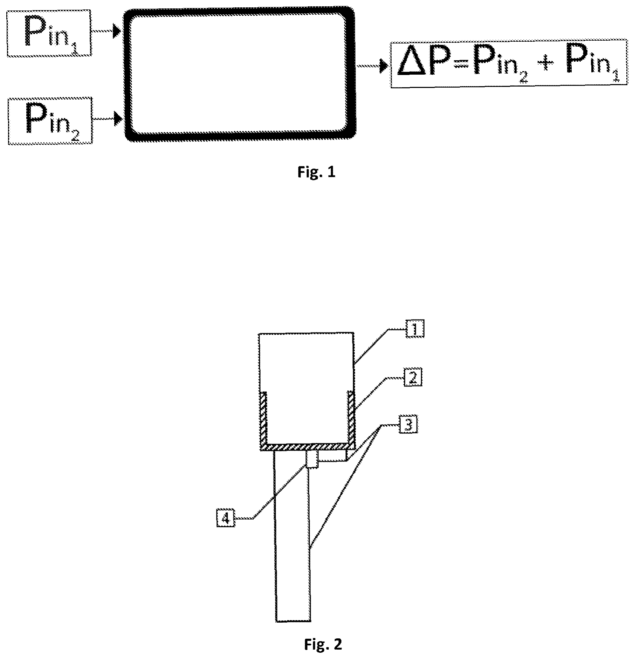

In order to obtain the value of the differential hydrostatic pressure in the liquid (*ΔP*) a differential pressure sensor is used, which measures the air or liquid pressure (Figure 1).

Considering a point in the top of the barrel (in the air pocket) and another in the end of a tube dived into the liquid, the hydrostatic pressure is transmitted to the sensor through air that stays "captured" inside the tight tubes connected to the differential pressure sensor. Therefore, the differential pressure sensor compares the pressure between the air pressure inside the barrel and the air pressure in the tube end, with possibility of the differential pressure sensor being situated at the top of the barrel, namely integrated in a stopper, which simplifies the device construction and operation, namely sparing two sensors.

The equation necessary for measuring the density, in the particular case of using two tubes with a ***Δh,*** is said Stevin's theorem. If the values of ***g*** and ***Δh*** are deemed constant and the value of ***ΔP*** is measured, it is possible to obtain the value of ***ρ**,* which in this case is the value of the liquid density.

The compensation of temperature causing the pressure of the air pocket to vary applies to both sides of the difference.

In an embodiment, if there is temperature variation inside the barrel, the air "captured" inside the measurement tubes expands, as a result of the temperature increase, or in opposition it contracts in case the temperature decreases. This leads to a variation of the hydrostatic pressure without a variation of the level, giving rise to a reading error.

This fact makes it necessary to acquire the temperature and compensate it for making the level measurement immune to that variation of the pressure inside the measurement tube.

In an embodiment, the "meniscus" effect is described and avoided, namely the weight thereof in the exact determination of the density, as well as other effects (dissolved gas). If the liquid inside the vessel releases gas (phenomenon occurring during the fermentation, resulting from the activity of yeasts), it may accumulate in the ends of the tube or tubes in case 2 tubes are dived in. The "meniscus" effect leads to the appearance of a "bubble" in the lower end of the measurement tube which releases itself whenever it has sufficient size for that to happen. This situation translates into a variation of the hydrostatic pressure without a variation of the level of the liquid or density thereof, thereby introducing a reading error. The bigger the "bubble" that can be accumulated by the "meniscus" effect, the bigger the introduced error. For minimising this error, the end of the tubes must have such a shape that it minimises the "meniscus" effect.

The present disclosure relates to a sensor device that may have the simultaneous function of vessel stopper or other format convenient to the installation, for measuring through a hydrostatic pressure difference, the volume or the density of a liquid contained in the vessel, wherein said sensor device comprises:
a main body;
a tube coupled to the main body for diving into the liquid when the vessel is covered or not with the sensor device;
a hydrostatic pressure differential sensor placed in the main body;
wherein the hydrostatic pressure differential sensor has two sensor inlets, a first inlet tight connected to the upper top of said tube so that the tube maintains air present in the interior thereof when dived into the liquid, and a second inlet for communicating with the interior of the vessel.

The present disclosure also relates to a sensor device of hydrostatic pressure difference for measuring volume or density of a liquid contained in a vessel, wherein said sensor device comprises:
a main body;
a tube for diving into the liquid;
a hydrostatic pressure differential sensor;
an air injector for injecting air into said tube;
wherein the tube is coupled to the main body;
wherein the hydrostatic pressure differential sensor has two inlets, a first inlet airtight connected to the upper top of said tube, configured so that the tube maintains air present in the interior thereof when dived into the liquid, and a second inlet for communicating with the interior of the vessel.

In an embodiment, it is foreseen the introduction of air into the measurement tubes (tube(s) for diving into the liquid), being that such air may be introduced through one or more air injectors, such as for example: air pumps, bottles with compressed air or any other pressurisation form.

The air injector allows for the air column and meniscus thereof to be reconstructed in a given measurement tube, before carrying out measure, for expelling the liquid that has introduced in the interior of the tube throughout time (for example, due to air leakages in the tube or in the pressure sensor); it also allows for expelling air from the tubes whenever necessary for removing any dirt, that has introduced in the tubes, through air injection.

In an embodiment, the sensor device may measure, through a hydrostatic pressure difference, the density of the liquid contained in the vessel wherein said sensor device may comprise:
a second tube coupled to the main body for diving into the liquid, being that the length of the second tube is different from the length of the first tube and
wherein the second inlet of the hydrostatic pressure differential sensor is airtight connected to the upper top of the second tube so that the second tube maintains air present in the interior thereof when dived into the liquid.

In an embodiment, the sensor device may measure the volume and density of the liquid contained in the vessel, being that the sensor device may comprise:
a second tube coupled to the main body for diving into the liquid, being that the length of the second tube is different from the length of the first tube and
a second hydrostatic pressure differential sensor;
wherein the second hydrostatic pressure differential sensor may have two inlets, a first inlet of the second differential sensor airtight connected to the upper top of the first tube, configured so that the tube maintains air present in the interior thereof when dived into the liquid, and a second inlet of the second differential sensor tight connected to the upper top of the second tube so that the second tube maintains air present in the interior thereof when dived into the liquid.

In an embodiment, the first or the second differential hydrostatic pressure differential sensor may comprise two non-differential pressure sensors and a differentiator for providing the difference between the pressures measured by the two non-differential sensors.

In an embodiment, the sensor device disclosed herein may comprise an additional tube for protecting the second inlet of the first hydrostatic pressure differential sensor.

In an embodiment, one or more of each tube, in particular of all the tubes, has an air injector for injecting air into the corresponding tube.

In an embodiment, the sensor device herein disclosed may comprise an electronic data processor connected to the hydrostatic pressure differential sensor and to the air injector, wherein said processor is configured for injecting air into said tube before obtaining a measure by the hydrostatic pressure differential sensor, in particular said processor is configured for injecting air into said tube immediately before obtaining a measure by the hydrostatic pressure differential sensor.

In an embodiment, the tube or tubes may have an inferior end angle-cut for avoiding or minimising meniscus effect of the liquid in the inferior end of the tube or tubes, in particular the inferior end may be angle-cut of 30-60°, preferably 45°.

In an embodiment, the tube or tubes may have an inferior end angle-cut for avoiding or minimising meniscus effect of the liquid in the inferior end of the tube or tubes.

In an embodiment, one or more tubes may have a superior end angle-coupled to the main body for avoiding or minimising the meniscus effect of the liquid in the inferior end of the tube or tubes.

In an embodiment, the tube or tubes may be stainless, teflon or plastic tube or tubes.

In an embodiment, the sensor device may comprise a temperature sensor coupled to the main body or to a said tube.

In an embodiment, the sensor device may comprise a sensor for measuring pH, turbidity, colour, sugar amounts or alcohol concentration of the liquid, or combinations thereof, coupled to the main body or to a said tube.

In an embodiment, the sensor device may be cylindrical, quadrangular section prismatic, rectangular section prismatic, or frusto-conical.

In an embodiment, the sensor device may comprise a wireless transmitter for transmitting data collected by the sensor or sensors of pH, turbidity, colour, sugar amounts or alcohol concentration of the liquid, or combinations thereof, coupled to the main body or to a said tube, being that the collected data may be transmitted periodically.

In an embodiment, the liquid may be wine, sparkling wine, juice, or a spirit drink.

In an embodiment, the vessel may be a barrel, tun, cask or vat.

In an embodiment, the sensor device herein disclosed may be a stopper of a said vessel, for example wherein the vessel may be a barrel, tun, cask or vat.

The present disclosure also relates to a barrel, a tun, a cask or vat that may comprise the sensor device disclosed herein.

The present disclosure further relates to a system for measuring volume and density of a liquid contained in vessels comprising a plurality of sensor devices herein disclosed.

In an embodiment, said system may comprise a data electronic processor that may be configured for receiving, processing and storing the data collected by the sensor device.

In an embodiment, said processor may generate and send an alert for at least a user via email or SMS.

Throughout the description and claims the word "comprises" and variations of the word "comprises", are not intended to exclude other technical features, as other components or steps. Additional objects, advantages and features of the disclosure will become clear to those skilled in the art upon examination of the description or may be learned by practice of the invention. The following examples and figures are for illustrating the description and should not be seen as limiting the scope of the disclosure. Furthermore, the present disclosure covers all possible combinations of specific or preferential embodiments herein described.

### Brief description of the drawings

For an easier understanding of the present disclosure figures are attached, which, represent preferred embodiments for illustrating the description and should not be seen as limiting the scope of the disclosure.
**Figure 1****:** Schematic representation of the operation of the differential sensor device used.
**Figure 2****:** Side view schematic representation of the set for measuring the level.
**Figure 3****:** Side view schematic representation of the set for measuring the density.
**Figure 4****:** Side view schematic representation of the set for measuring the level and density.
**Figure 5****:** Side view schematic representation of the set for measuring the level placed in the barrel.
**Figure 6****:** Side view schematic representation of the set for measuring the density placed in the barrel.

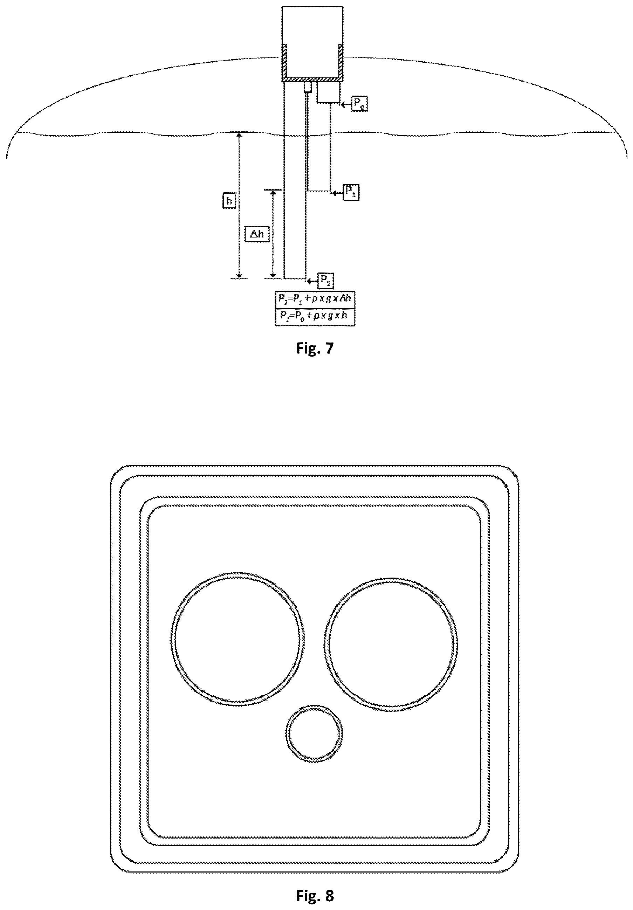

**Figure 7****:** Side view schematic representation of the set for measuring the level and density placed in the barrel.
**Figure 8****:** Bottom view schematic representation of the set for measuring the level or density.

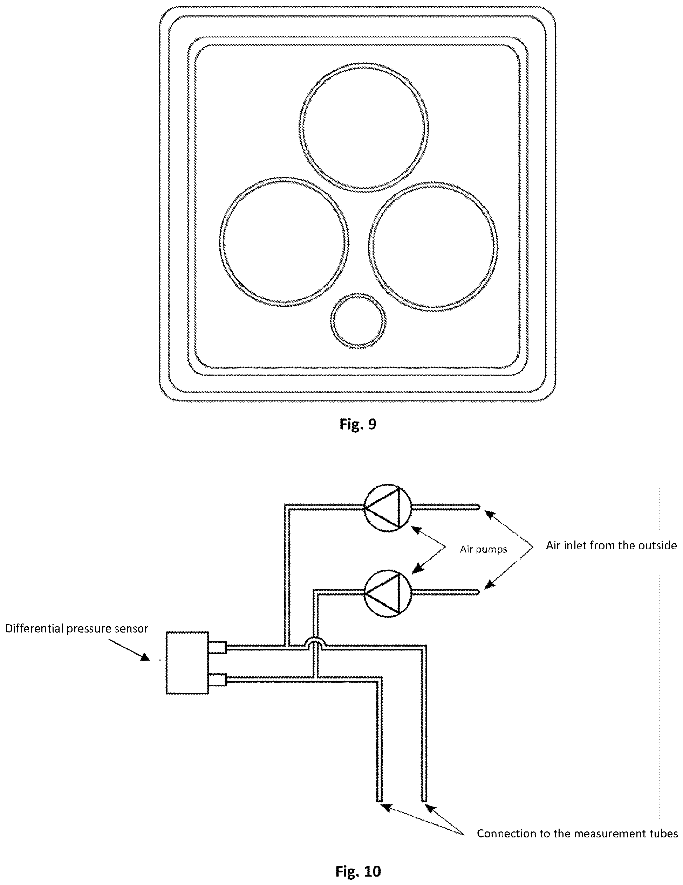

**Figure 9****:** Bottom view schematic representation of the set for measuring the level and density.
**Figure 10****:** Schematic representation of the system with pumps for introducing air or air injectors.

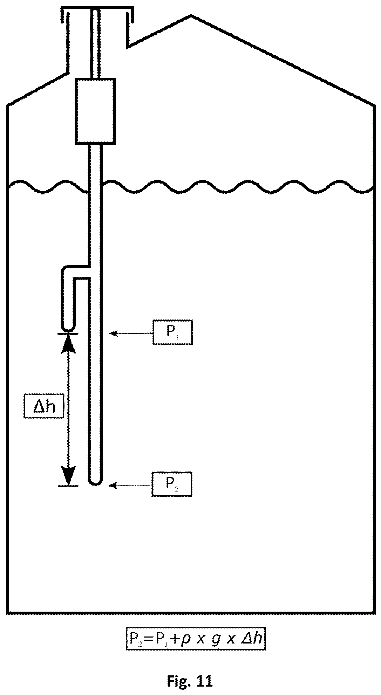

**Figure 11****:** Side view schematic representation of the set for measuring the density placed in the vat.
**Figure 12****:** Schematic representation of the embodiment for preventing the "meniscus" effect by the angle-cut of the inferior end of the measurement tube.

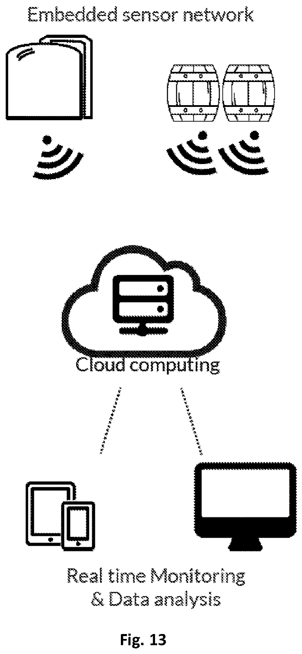

**Figure 13****:** Schematic representation of the integrated system herein disclosed.

### Detailed description

**Figure 2** represents an embodiment for measuring the level of the liquid contained in the barrel, disclosing some of the components comprised in the present disclosure. **Figure 2** represents in particular a side view of the present disclosure wherein 1 represents the carcass/main body of the stopper/sensor device, 2 represents a silicone joint, that may exist or not, on accumulating stopper function (tightness) or not of the vessel; 3 represents two tubes connected to the sensor, one for measuring the air pocket pressure and another with air in the interior thereof for measuring the liquid pressure at a given depth, in particular tubes that may comprise in the composition thereof a material compatible with the liquid with which stainless steel, teflon or plastic is in contact; and 4 represents a temperature sensor.

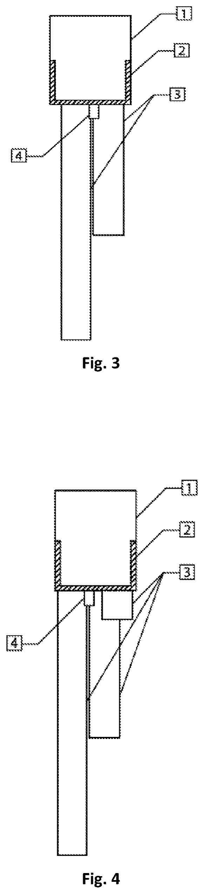

**Figure 3** represents an embodiment, in particular it represents a side view of the set disclosed herein for measuring the liquid density contained in the barrel, with two tubes connected to the sensor with air in the interior thereof for measuring the liquid pressure at two predetermined depths.

**Figure 4** represents an embodiment of the present disclosure, in particular it represents a side view of the set for measuring the liquid level and density contained in the barrel, with three tubes connected to the sensor, one for measuring the air pocket pressure and two tubes with air in the interior thereof for measuring the liquid pressure at two predetermined depths.

The tube for measuring the pressure in the air pocket in the described embodiments is optional, since the sensor will function equally well without it. The tube has here a physical protection function of the sensor.

The temperature sensor is optional, in case the temperature measure is not used or is provided by another sensor, inside or outside the barrel.

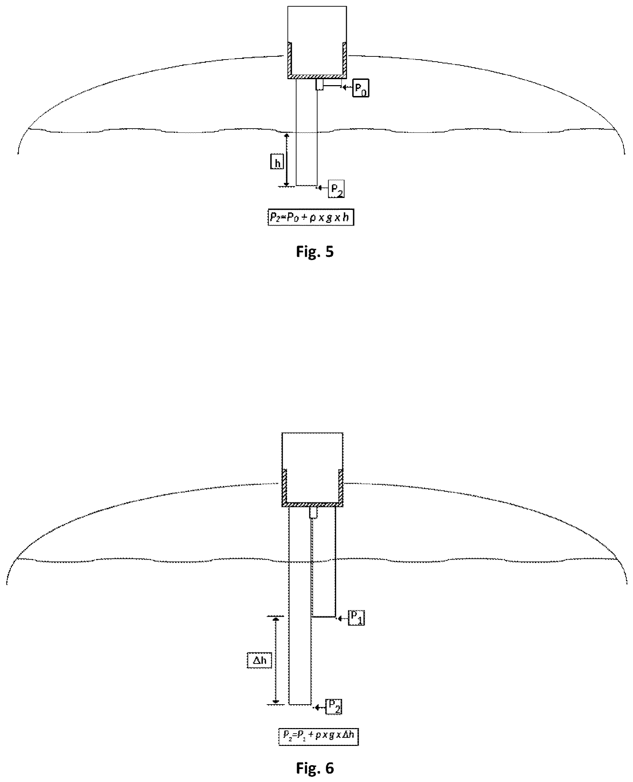

**Figure 5** represents an architecture of the monitoring platform, in particular a side view of the measurement set in the level placed in the barrel, being that **P₀** represents the hydrostatic pressure measured in the interior of the vessel; **P₂** represents the hydrostatic pressure measured in the tube end (first tube) that is dived into the liquid contained in the barrel and h represents the height between the point where the measurement of **P₂** is made and the surface of the liquid.

**Figure 6** represents the architecture of the monitoring platform in particular a side view of the set for density measurement placed in the barrel wherein **P₂** represents the hydrostatic pressure measured in the tube end (first tube) that is dived into the liquid contained in the barrel, **P₁** represents the hydrostatic pressure measured in the end of a second tube that is dived into the liquid contained in the barrel and **Δh** represents the height difference between the points **P₁** and **P₂.** **Figure 6** represents a joint embodiment of the level and density sensor.

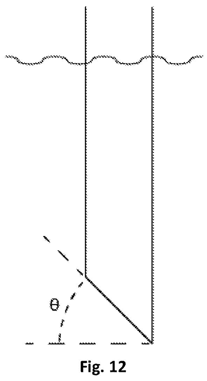

**Figure 12** represents an embodiment allowing for reducing or minimising the meniscus effect that may introduce reading errors, either of liquid level or density level. Therefore for avoiding or minimising the meniscus effect, each tube may comprise in the inferior end thereof an angle-cut, for example between 30-60°, preferably 45°, being that this angle is measured between the inferior opening plan of the tube and the liquid surface. Alternatively or complementarily, the tube may be tilted in relation to the vertical.

In an embodiment, the initial calibration may be carried out by the following steps:
carrying out a measure with the sensor outside the liquid, that is in the air;
carrying out a measure with the sensor dived into the liquid d/2 mm (where d represents the total length of the sensor in mm, that is, it represents the maximum value of the level the sensor can measure);
carrying out a measure with the sensor dived into the liquid d mm (where d represents the maximum value of the level the sensor can measure).

In an embodiment, the initial calibration may also be carried out by the following steps:
carrying out a measure with the sensor outside the liquid, that is in the air;
carrying out a measure with the sensor dived into the liquid d/3 mm (where d represents the maximum value of the level the sensor can measure);
carrying out a measure with the sensor dived into the liquid 2 x d/3 mm (where d represents the maximum value of the level the sensor can measure);
carrying out a measure with the sensor dived into the liquid d mm (where d represents the maximum value of the level the sensor can measure).

In an embodiment, the initial calibration carried out in 4 steps is a more precise calibration than the calibration carried out in 3 steps because more points are obtained giving rise to a better adjustment equation, decreasing the error associated to the calibration.

Although merely particular embodiments of the present disclosure have been represented and described herein, those skilled in the art will know how to introduce modifications and replace some technical features with equivalent ones, depending on the requisites of each situation, without departing from the scope of protection defined by the appended claims. The term "comprises" or "comprising" when used in this document is intended to indicate the presence of the mentioned features, elements, integers, steps and components, but not to preclude the presence or addition of one or more other features, integers, steps and components or combinations thereof. The following claims additionally set out embodiments of the disclosure.

The present application is a divisional application of EP17742854.7, the disclosure of which is herewith explicitly incorporated by reference into the present disclosure for all purposes.
In the following, a set of items is disclosed. The items are numbered to facilitate referencing to the features of one item in other items. The items form part of the disclosure of the present application and could be made subject to independent and/or dependent claims irrespective of what currently is claimed in the application. We note, however, that the scope of protection is defined by the appended claims, where the following items do not constitute claims. The items are:
1. Sensor device for measuring volume and/or density of a liquid contained in a vessel, wherein said sensor device comprises:
   a main body;
   a tube for diving into the liquid;
   a hydrostatic pressure differential sensor;
   an air injector for injecting air into said tube;
   wherein the tube is coupled to the main body;
   wherein the hydrostatic pressure differential sensor has two entries, a first inlet airtight connected to the upper top of said tube, configured so that the tube maintains air present in the interior thereof when dived into the liquid, and a second inlet for communicating with the interior of the vessel.
2. Sensor device for measuring the density of the liquid contained in the vessel, according to the previous item, comprising:
   a second tube coupled to the main body for diving into the liquid, with a length different from the first tube;
   wherein the second inlet of the hydrostatic pressure differential sensor is airtight connected to the upper top of the second tube so that the second tube maintains air present in the interior thereof when dived into the liquid.
3. Sensor device for measuring the volume and density of the liquid contained in the vessel, according to item 1, comprising:
   a second tube coupled to the main body for diving into the liquid, with a length different from the first tube;
   a second hydrostatic pressure differential sensor;
   wherein the second hydrostatic pressure differential sensor has two inlets, a first inlet of the second differential sensor airtight connected to the upper top of the first tube, configured so that the tube maintains air present in the interior thereof when dived into the liquid, and a second inlet of the second differential sensor airtight connected to the upper top of the second tube so that the second tube maintains air present in the interior thereof when dived into the liquid.
4. Sensor device according to any of the previous items wherein a hydrostatic pressure differential sensor comprises two non-differential pressure sensors and a differentiator for providing the difference between the pressures measured by the two non-differential sensors.
5. Sensor device according to items 1 and 3-4 comprising an additional tube for protecting the second inlet of the first hydrostatic pressure differential sensor.
6. Sensor device according to any of the previous items comprising an electronic data processor connected to the hydrostatic pressure differential sensor and to the air injector, wherein said processor is configured for injecting air into the tube before obtaining a measure by the hydrostatic pressure differential sensor, in particular said processor is configured for injecting air into the tube immediately before obtaining a measure by the hydrostatic pressure differential sensor.
7. Sensor device according to any of the previous items wherein the tube or tubes have a lower end angle-cut for avoiding meniscus effect of the liquid in the lower end of the tube or tubes.
8. Sensor device according to any of the previous items wherein one or more tubes have an upper end angle-coupled to the main body for avoiding the meniscus effect of the liquid in the lower end of the tube or tubes.
9. Sensor device according to any of the previous items wherein the tube or tubes are stainless, teflon or plastic tube or tubes.
10. Sensor device according to any of the previous items comprising a temperature sensor coupled to the main body or to a said tube.
11. Sensor device according to the previous items wherein said sensor further comprises a sensor for measuring pH, turbidity, colour, sugar amounts or alcohol concentration of the liquid, or combinations thereof, coupled to the main body or to a said tube.
12. Sensor device according to the previous items wherein said sensor is cylindrical, prismatic with a quadrangular section, prismatic with a rectangular section, or frusto-conical.
13. Sensor device according to any of the previous items comprising a wireless transmitter for transmitting data collected by the sensor or sensors.
14. Sensor device according to the previous item wherein the collected data are transmitted periodically.
15. Sensor device according to any of the previous items wherein the liquid is wine, sparkling wine, juice, or spirit drink.
16. Sensor device according to the previous items wherein the vessel is a barrel, tun, cask or vat.
17. Sensor device according to any of the previous items wherein the sensor is a stopper of said vessel.
18. Sensor device according to the previous item wherein the vessel is a barrel, tun, cask or vat.
19. Barrel, tun, cask or vat comprising the sensor device of any of the previous items.
20. System for measuring volume and density of a liquid contained in vessels comprising a plurality of sensor devices of one or more items 1-18.
21. System according to the previous item comprising an analogue or digital electronic processor of data and wherein said processor is configured for receiving, processing and storing the data collected by the sensor device.
22. System according to the previous item wherein the processor generates a sound or visual alarm, or generates and sends an alert for at least a user via email, push notification or SMS according to the measured volume and/or density of liquid.

## Claims

1. A sensor device for measuring a volume of a liquid contained in a vessel, the sensor device comprising:
a main body;
a first stainless-steel tube coupled to the main body and configured to be submerged in the liquid;
an air injector configured to inject air into the first stainless-steel tube; and
a hydrostatic pressure differential sensor having a first inlet and a second inlet, wherein:
the first inlet is airtight-connected to the first stainless-steel tube such that the first stainless-steel tube maintains air in an interior thereof when the first stainless-steel tube is inserted into the liquid,
the second inlet is configured to communicate with air in an interior of the vessel, and
the first stainless-steel tube is straight, is tilted relative to vertical to avoid meniscus effects, and is angle-cut.

2. The sensor device of claim 1, wherein the hydrostatic pressure differential sensor comprises two non-differential pressure sensors and a differentiator configured to output a pressure difference.

3. The sensor device of claim 1, comprising a processor coupled to the air injector and to the hydrostatic pressure differential sensor, the processor being configured to inject air into the first stainless-steel tube immediately before obtaining a measurement.

4. The sensor device of claim 1, comprising an additional tube configured to protect the second inlet of the hydrostatic pressure differential sensor.

5. The sensor device of claim 1, comprising a temperature sensor coupled to the main body or to the first stainless-steel tube.

6. The sensor device of claim 1, wherein the device comprises a sensor for measuring at least one of: pH, turbidity, colour, sugar amount, or alcohol concentration.

7. The sensor device of claim 1, wherein the sensor device is cylindrical, prismatic with a quadrangular section, prismatic with a rectangular section, or frusto-conical.

8. The sensor device of claim 1, wherein the sensor device comprises a wireless transmitter configured to transmit data collected by the hydrostatic pressure differential sensor.

9. The sensor device of claim 1, wherein the collected data are transmitted periodically.

10. The sensor device of claim 1, wherein the liquid is wine, sparkling wine, juice, or a spirit drink.

11. The sensor device of claim 1, wherein the sensor device is a stopper of the vessel.

12. The sensor device of claim 1, wherein the vessel is a barrel, tun, cask, or vat.

13. The sensor device of claim 1, wherein the hydrostatic pressure differential sensor is configured to determine a density of the liquid contained in the vessel.

14. The sensor device of claim 1, comprising a second stainless-steel tube coupled to the main body and having a length different from the first stainless-steel tube.

15. The sensor device of claim 14, comprising a second hydrostatic pressure differential sensor having a first inlet airtight-connected to the first stainless-steel tube and a second inlet airtight-connected to the second stainless-steel tube.
